(19) Europäisches Patentamt
European Patent Office
Office européen des brevets

(11) **EP 0 536 858 B1**

(12) **EUROPÄISCHE PATENTSCHRIFT**

(45) Veröffentlichungstag und Bekanntmachung des Hinweises auf die Patenterteilung:
**24.07.1996 Patentblatt 1996/30**

(51) Int. Cl.$^6$: **A61N 1/365**, A61M 1/12

(21) Anmeldenummer: **92250257.0**

(22) Anmeldetag: **14.09.1992**

(54) **Stimulationssystem für einen Skelettmuskel**

Stimulation system for a skeletal muscle

Système de stimulation pour un muscle du squelette

(84) Benannte Vertragsstaaten:
**DE FR GB IT NL SE**

(30) Priorität: **12.09.1991 DE 4130596**

(43) Veröffentlichungstag der Anmeldung:
**14.04.1993 Patentblatt 1993/15**

(73) Patentinhaber: **BIOTRONIK Mess- und Therapiegeräte GmbH & Co Ingenieurbüro Berlin D-12359 Berlin (DE)**

(72) Erfinder: **Schaldach, Max, Prof. Dr. W-8520 Erlangen (DE)**

(74) Vertreter: **Christiansen, Henning, Dipl.-Ing. Patentanwalt Pacelliallee 43/45 14195 Berlin (DE)**

(56) Entgegenhaltungen:
**WO-A-91/08006**

## Beschreibung

Die Erfindung betrifft ein Stimulationssystem der im Oberbegriff des Anspruchs 1 angegebenen Art.

Die schwere therapierefraktäre Herzinsuffizienz - aufgrund ihrer Verbreitung und Mortalität ein bedeutendes Problem - ist mit der Technik der Kardiomyoplastie heilbar.

In der Herzchirurgie besteht generell ein großer Bedarf an Systemen, die die Pumpfunktion der Herzens ganz oder teilweise übernehmen können. Die Kardiomyoplastie - diese vielversprechende Methode, die sich seit 1985 in klinischer Anwendung befindet - ist eine Technik, den Verlust von Herzmuskelgewebe funktionell durch Skelettmuskulatur, insbesondere durch den Muskulus latissimus dorsi, zu ersetzen. Es sind bereits solche Operationen an Herzpatienten zur Unterstützung der Herzkontraktion bekannt, wo ein körpereigener, vorzugsweise aus der Rückengegend stammender Skelettmuskel der quergestreiften Muskulatur ganz oder teilweise um das Herz gelegt wird.

Es ist ebenfalls bekannt, daß dieser Skelettmuskel durch eine 6-wöchige chronische Elektrostimulation mit niedrigen Frequenzen um 2 bis 10 Hz trainiert werden kann und dann relativ ermüdungsresistent ist. Dabei erfolgt eine Umwandlung des an sich mehrere Muskelfaserarten aufweisenden Muskelgewebes derart, daß die ermüdungsresistentere am Ende der Konditionierung überwiegt.

Es wurden solche Stimulationssysteme entwickelt, die Einzelimpulse in kurzer Folge abgeben (Burst-Stimulatoren). Da der durch einen Einzelimpuls stimulierte Skelettmuskel latissimus dorsi (LDM) mit einer ca. 70 ms andauernden Kontraktion reagiert, die Zeitdauer der Herzsystole jedoch 200 bis 250 ms dauert, wird die Muskelkontraktion an den Zeitverlauf der Herzkontraktion elektronisch angepaßt. Entsprechend der Burstdauer verlängert sich dann die Kontraktionsdauer. Ein derartiger bekannter implantierbarer Schrittmacher ist zweikanalig aufgebaut, wobei jeder Kanal über ein Empfangsteil, ein Synchronisierteil und ein Ausgangsteil verfügt.

Aus den Druckschriften WO-A-91/08006 oder WO-A-91/08021 ist jeweils ein gattungsgemäßes Stimulationssystem für einen Skelettmuskel zur Unterstützung der Herztätigkeit bekannt, das auch eine Anpassung der Muskelstimulation an unterschiedliche Belastungszustände erlaubt.

Die Variationsbreite der Stimulationsintensität bzw. einzelner Stimulationsparameter und insbesondere die Freiheitsgrade des Zusammenwirkens des Muskelstimulators mit einem gleichzeitig vorhandenen Herzschrittmacher sind jedoch hier noch begrenzt.

Der Erfindung liegt damit die Aufgabe zugrunde, einen zur Unterstützung des Herzmuskels herangezogenen Skelettmuskel (Musculus latissimus dorsi) derart zu stimulieren, daß er die gleiche Funktion wie der Herzmuskel erfüllen kann.

Diese Aufgabe wird mit den im Anspruch 1 angegebenen Maßnahmen gelöst.

Die Erfindung beruht auf der Erkenntnis, daß nicht nur die aus dem Ausgangssignal des Sensors und/oder aus dem Herzrythmus abgeleitete Escapesequenz, sondern auch die Stimulationsintensität eng an den tatsächlichen aktuellen physiologischen Bedarf des Patienten angepaßt sein muß. Dabei muß die Stimulation des Skelettmuskels an die besondere Art der Reizleitung angepaßt sein.

Nach der Erfindung wird damit für einen um das Herz des Patienten gelegten, die Herzkontraktion zusätzlich unterstützenden Skelettmuskel ein Stimulationssystem angegeben, das aus den Ausgangssignalen eines geeigneten physiologischen Herzschrittmachers solche Stimulationsimpulse ableitet, die dem tatsächlichen aktuellen physiologischen Bedarf des Patienten entsprechen.

Hierbei erfolgt insbesondere unter Erfassung des Zustandes des autonomen (bzw. vegetativen) Nervensystems und von daraus abgeleiteten Informationen die Stimulation, Reizung bzw. Aktivierung des zur Unterstützung des Herzmuskels herangezogenen Skelettmuskels so abgestuft, daß sie auf die physiologischen Bedürfnisse des Gesamtorganismus in hämodynamischer und metabolischer Hinsicht abgestimmt ist.

In vorteilhafter Weise erfolgt die Stimulation des Skelettmuskels durch einen elektrischen Impulsgeber mit einer Impulsserie, deren reizwirksame Parameter durch die aus dem Zustand des autonomen Nervensystems direkt oder indirekt abgeleiteten Größen in der nachfolgend beschriebenen Weise eingestellt werden.

Die Beaufschlagung der mit dem Skelettmuskel verbundenen Elektroden, erfolgt über den Stimulationsteil und durch den Schrittmacher dabei einerseits mit einer bestimmten automatisch einstellbaren Rate R der Stimulationsimpulse und andererseits entsprechend einer für die aktuelle körperliche Belastung bzw. den kardiovaskulären Bedarf repräsentativen Größe mit einer Stimulationsleistung, insbesondere Amplitude und Dauer und/oder mit einer veränderten Zahl der jeweils angesteuerten Elektroden.

Bei intaktem Herzeigenrhythmus wird dabei die für die aktuelle körperliche Belastung bzw. den kardiovaskulären Bedarf repräsentative Größe durch dessen Herzrate gebildet.

Bei fehlendem Herzeigenrhythmus werden Stimulationsimpulse mit definierter Rate vom Herzschrittmacher erzeugt.

Wenn die Herzrate einen bestimmten programmierbaren Wert überschreitet, wird das Verhältnis von Herzaktion zu Muskelkontraktion verändert und die an die Elektroden abgegebene Impuls-Leistung erhöht. In Abhängigkeit von der für die aktuelle körperliche Belastung oder den kardiovaskulären Bedarf repräsentativen Größe wird eine Untersetzung der Rate in einem von dieser Belastung abhängigen Verhältnis zur natürlichen - oder bei Ausbleiben des herzeigenen Rhythmus - zum durch den herkömmlichen Schrittmacher stimulierten Rhythmus vorgenommen, wobei das Untersetzungsverhältnis mit zunehmender körperlicher Belastung bzw. kardiovaskulärem Bedarf bis zum Verhältnis 1:1 abnimmt.

Seitens des Herzschrittmachers wird die für die aktuelle körperliche Belastung repräsentative Größe bei fehlendem Herzeigenrhythmus aus einem Sensorsignal für die Aktivität des Patienten oder einer sekundären für die Kreislaufbelastung repräsentative Größe, wie der Bluttempertur, der Blutsauerstoffsättigung oder dem pH-Wert des Blutes, erzeugt.

Als weitere physiologische Kontrolle der Stimulationsintensität, eignen sich dabei solche Größe, die unmittelbar ein Maß für den internen cardiovaskulären Herzleistungsbedarf bildet und nicht - wie die von Aktivität des Patienten gesteuerten Schrittmacher - den Leistungsbedarf über aus einer externen Größe herleitet.

Die Analyse der Kreislaufhämodynamik ergibt, daß die kardiovaskuläre Leistung in der Form eines »negative feedback controlled system" ausreichend beschrieben werden kann.

Der durchschnittliche arterielle Blutdruck ist die kontrollierte Größe und der Herzleistungsbedarf die kontrollierende Variable. Unter physiologischen Bedingungen funktioniert die kurzfristige Steuerung des mittleren arteriellen Blutdrucks gut. Alle Anforderungen an das kardiovaskuläre System werden im Gleichgewicht gehalten und liefern daher eine zuverlässige Perfusion des Gewebes.

Die meisten Anforderungen an das kardivaskuläre System beeinflussen den peripheren Gefäßwiderstand. Die relevantesten dieser Anforderungen sind: körperliche Betätigung, Haut- und die Kerntemperatur des Körpers, der Säurespiegel, das Gleichgewicht und die Körperhaltung. Eine der Anforderungen beeinflußt sowohl die resistiven als auch die kapazitiven Eigenschaften der Peripherie durch Veränderungen in der Verengung der Arteriola oder Venula. Die sekundären Wirkungen sind Veränderungen im Gefäßwiderstand, im Blutdruck, im Venenrückfluß und in der Herzleistung. Beispielsweise kann sich der Gefäßwiderstand während einer körperlichen Betätigung um das Fünffache verändern. Um den Blutdruck konstant zu halten, müssen der Venenrückfluß und die Herzleistung entsprechend mit entgegengesetzter Wirkung verändert werden.

Der Herzleistungsbedarf ist die kontrollierende Größe. Sie ist das Produkt der Herzfrequenz und des Schlagvolumens.

Die Veränderungen des Herzleistungsbedarfs stehen im umgekehrten Verhältnis zu den Veränderungen des Gefäßwiderstandes mit dem Ergebnis, daß der mittlere arterielle Blutdruck im wesentlichen konstant gehalten wird. Unter physiologischen Bedingungen wird die Anpassung des Herzleistungsbedarfs an die Veränderungen des Gefäßwiderstandes von einem »closed-loop feedback" mit Barorezeptoren im Aortenbogen und im Sinus caroticus, die als Drucksensoren arbeiten, durchgeführt. Die Ausgangssignale dieser Barorezeptoren werden den medullären kardiovaskulären Zentren zugeführt. Von diesen Zentren werden Ausgangssignale zum Herz über den Sympathikus oder Parasympathikus geleitet.

Der Sympathikus reguliert die Sinusfrequenz und das ventrikuläre Schlagvolumen. Der Parasympathikus dagegen beeinflußt überwiegend die Herzfrequenz. Die Kombination der chrontropischen und der inotropischen Faktoren führen zusammen zur Steuerung der Herzleistung. Andere Variable, die das Herz aufgrund der gleichen externen Pfade beeinflussen sind u.a.: Schmerz, Emotion und physiologischer Streß.

Bei Patienten mit einer chronotropischen Insuffizienz wird der Rückkopplungsmechanismus, mit dem die Herzfrequenz über den Sinusknoten reguliert wird, unterbrochen. Als Folge können diese Patienten nur durch eine Änderung des Schlagvolumens auf Belastungen reagieren. Die mögliche Schlagvolumenveränderung ist aber bei einer körperlichen Betätigung sehr gering.

Die chronotropische Komponente bei der Herzleistungsreserve kann wiederhergestellt werden, indem die natürliche Stimulationsvorrichtung durch eine künstliche ersetzt wird.

Die Stimulationstheorie bietet einige verschiedene Strategien an, um den Einfluß einer Belastung des Kreislaufs entgegenzuwirken.

1) Open-loop control wird bei vielen frequenzadaptiven Herzschrittmachern angewandt;

2) Die Leistungsforderung steht im Bezug zur Belastung, in dem die Herzleistung an die berechnete Leistungsforderung angepaßt wird. Temperatur- und atemgesteuerte Herzschrittmacher gehören dieser Kategorie an.

3) Physiologisch gesteuert, wobei das gemessene Signal den Kreislaufbedarf direkt widerspiegelt.

Eine zentrale, für den kardiovaskulären Bedarf maßgebliche und gut reproduzierbare Größe des autonomen Nervensystems (ANS) stellt die regionale effektive Steigung (ROI) der intrakardialen Impedanz des rechten Ventrikels dar. Hierbei wird bevorzugt das Intervall mit der regelmäßig größten Änderung bei isovolumetrischer Kontraktion und hiervon wiederum der Bereich mit der größten Veränderung bei Belastungsänderungen des Patienten ausgewählt. Damit hat das ermittelte Signal brauchbare Eigenschaften, die dazu führen, daß es sich bevorzugt als Steuersignal für die das Herzzeitvolumen bestimmenden Größen des Herzschrittmachers eignet.

Die regionale effektive Steigung steht im umgekehrten Verhältnis zur Myokardkontraktilität. Je größer die Kontraktilität, desto schneller muß sich der notwendige Druck zur Öffnung der ventrikulären Herzklappen aufbauen kann.

Die ANS-Größe bildet dabei eine von weiteren Einflußgrößen weitgehend unabhängige, für den Herzleistungsbedarf repräsentative autonome Nervenaktivitätsgröße. Sie zeigt inotropisches Verhalten als Anzeichen für den cardiovaskulären Bedarf als Steuersignal für die Wiederherstellung des chronotropischen Verhaltens und stellt eine für die Modulation der Kontraktilität maßgebliche Größe dar.

Insbesonders ist die Ventrikulärkontraktilität unter sympathischer Kontrolle. Da die Vorinjektionsphase in Beziehung zur Kontraktilität steht, können Veränderungen in ANS verwendet werden, um Veränderungen im sympatischen Tonus verfolgen zu können.

Da die Kontraktilität des linken Ventrikels Teil des Kreislaufbereichs mit höherem Druck ist, wird ANS mit Bezug auf diese Kammer definiert. Dagegen eignet sich aber der rechte Ventrikel besser für intrakardiale Messungen der ANS-Größe.

ANS, die im rechten und im linken Ventrikel während einer körperlichen Betätigung gemessen worden sind, weisen eine hervorragende Beziehung zueinander auf, obwohl die ANS-Größe des linken Ventrikels etwas länger als die ANS-Größe des rechten Ventrikels ist.

Die Aufgabe einen Herzschrittmacher zu konstruieren, der ANS-Größe als Kontrollsignal verwendet, verwandelt sich in die Aufgabe, eine ANS-bezogene Größe mit hinreichender Genauigkeit messen zu können. Eine Methode basiert auf intrakardialen Impedanzmessungen. Es hat sich gezeigt, daß das intraventrikuläre Volumen durch die Impedanz meßbar ist. Diese Messungen wurden mit einer Anordnung von Multielektroden im entsprechenden Ventrikel durchgeführt. Um den Endpunkt der ANS zu ermitteln, ist es nicht notwendig, den absoluten Wert des Volumens zu messen. Es genügt, wenn man den Zeitpunkt ermittelt, von dem an der Volumen sich zu ändern beginnt. Es hat sich gezeigt, daß eine ausreichende Auflösung mit einer im Ventrikel angeordneten unipolaren Elektrode erzielt werden kann, wobei das Herzschrittmachergehäuse als Gegenelelektrode dient.

Bei der für den kardiovaskulären Bedarf repräsentativen Größe handelt es sich somit um eine durch von weiteren Einflußgrößen weitgehend unabhängige autonome Nervenaktivitätsgröße. Diese wird durch Impedanzplethysmographie gewonnen. Es handelt sich bei der autonomen Nerveninformation um die regionale effektive Steigung der Leitfähigkeit im rechten Ventrikel, insbesondere im Bereich der maximalen Leitfähigkeitsänderung während isovolumetrischer Kontraktion, wobei dieser Bereich mit einem Zeitfenster ausgewählt wird. Bevorzugt wird ein Bereich, in dem sich bei unterschiedlicher Belastung eine maximale Leitfähigkeitsänderung feststellen läßt.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Fig. 1 ein Blockschaltbild eines vorteilhaften Ausführungsbeispiels des erfindungsgemäßen Stimulationssystems sowie

Fig. 2 ein Blockschaltbild der Signalverarbeitungsschaltung des Systems nach Figur 1.

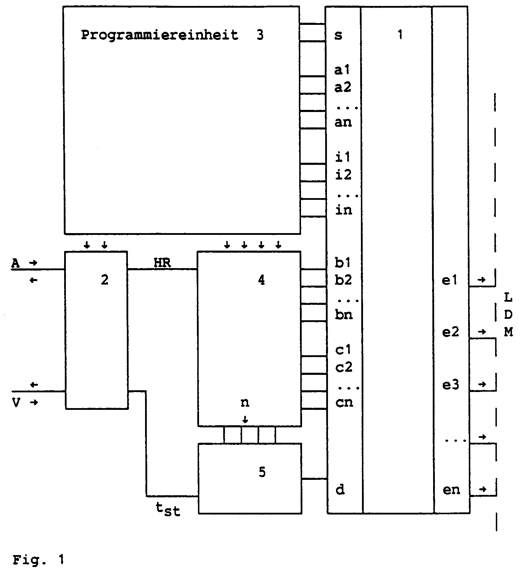

Bei dem in Figur 1 gezeigten Stimulationssystem werden von einer Signalverarbeitungsschaltung 1 Stimulationsimpulse erzeugt, die aus den Ausgangssignalen HR und $t_{st}$ eines Herzschrittmachers 2 detektiert werden. Das Ausgangssignal für die Herzrate HR wird dabei einer programmierbaren Auswerteeinheit 4, die insbesondere als Decoder ausgeführt ist, und das Ausgangssignal für den Stimulationszeitpunkt $t_{st}$ wird einem einstellbaren Synchronisierteil 5, das insbesondere als Dividierer ausgebildet ist, zugeführt. Außerdem ist eine Programmiereinheit 3 mit dem Herzschrittmacher 2, mit dem Decoder 4 und mit der Signalverarbeitungsschaltung 1 verbunden, welche jeweils programmierbar sind und entsprechende programmiereingänge aufweisen. Durch eine Steuereinheit 6 ist die Signalverarbeitungsschaltung 1 ein- und ausschaltbar.

Die für die aktuelle körperliche Belastung bzw. den kardiovaskulären Bedarf repräsentative Größe wird bei intaktem Herzeigenrhythmus durch dessen Herzrate HR und bei fehlendem Herzeigenrhythmus durch eine vom Herzschrittmacher 2 erzeugte Herzrate HR gebildet.

Der programmierbare Decoder 4 gestattet die Ableitung von verschiedenen Faktoren n aus der Herzrate HR. Dabei entspricht eine Division durch den Faktor n = 1 der beispielsweise vom Medtronic Synchron Schrittmacher her bekannten Steuerung bzw. der durch ein natürliches Signal hervorgerufenen Stimulierung. In Abhängigkeit von der für die aktuelle körperliche Belastung oder den kardiovaskulären Bedarf repräsentativen Größe HR erfolgt eine Untersetzung der Herzrate in einem von dieser Belastung abhängigen Verhältnis zur natürlichen - oder bei Ausbleiben des herzeigenen Rhythmus - zum durch den herkömmlichen Schrittmacher stimulierten Rhythmus, wobei das Untersetzungsverhältnis mit zunehmender körperlicher Belastung bzw. kardiovaskulärem Bedarf bis zum Verhältnis 1:1 abnimmt.

Der Decoder 4 weist Ausgänge zur Anwahl und Ansteuerung der Elektroden mit der erforderlichen Leistung auf.

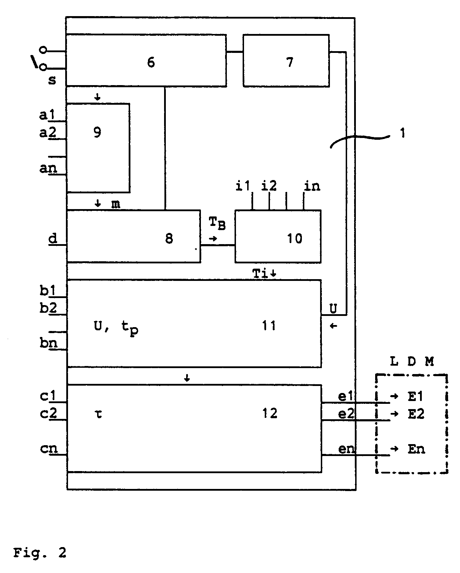

Im Blockschaltbild gemäß Figur 2 ist die programmierbare Signalverarbeitungsschaltung 1 dargestellt.

Die ein- und ausschaltbare Steuereinheit 6 ist einerseits mit einer Spannungsverdopplerschaltung 7 und andererseits direkt und über eine Programmiereingänge $a_1$ bis $a_n$ aufweisende Schaltung zur Burstlängeneinstellung 9 indirekt mit dem Burst-Timer 8 verbunden. Dieser zählt entsprechend der Voreinstellung der Burstlänge $T_B$ bei Ansteuerung durch den Dividierer 5 bis zu 8 Impulse. Diese vom Dividierer abgegebenen und für die Erzeugung einer mit der Herzstimulierung synchronisierten Burstdauer $T_B$ erforderlichen Impulse sind durch den vom Herzschrittmacher abgegebenen Triggerimpuls im Stimulationszeitpunkt $t_{st}$ synchronisiert und liegen an mindestens einem Eingang d des Burst-Timers 8 an.

Der Ausgang des Burst-Timers 8 ist mit dem Steuereingang eines programmierbaren Start-Stop-Oszillators 10 verbunden, der entsprechend dem von der Schaltung 9 vorgegebenen Stimulationsmodus, d.h. während der Burstdauer $T_B$, kurze Impulse mit einer Zeit-

dauer von $T_p$ = 0,2 bis 1 ms bei einer Erregungsfrequenz f = 15 bis 40 Hz abgibt. Eine Veränderung des Impulsintervalls $T_i$ = 1 / f über mit der Programmiereinheit 3 verbundene Programmiereingänge $i_1$ bis $i_n$ beeinflußt die Kontraktionskraft über eine Änderung der Erregungsfrequenz, damit auch der Kraft, die von einer einzelnen Motoreinheit erzeugt wird (Temporal Summation).

Die Spannungsverdopplerschaltung 7 erzeugt vorzugsweise - 5,6 V als Betriebsspannung für eine Leistungsstufe 11 aus der Speisespannung Us = 2,8 V vorhandener geeigneter Schrittmacher. Diese Leistungsstufe 11 verstärkt die Leistung der Ausgangsimpulse des Start-Stop-Oszillators 10 in Abhängigkeit von einer über die Steuereingänge $b_1$ bis $b_n$ automatisch erfolgenden programmierbaren Einstellung. Die Steuereingänge $b_1$ bis $b_n$ sind hierzu mit den entsprechenden Ausgängen des programmierbaren Decoders 4 verbunden. Bei erhöhter Aktivität wird das Herz durch den Skelettmuskel verstärkt unterstützt, denn eine Erhöhung der Impulsbreite $T_i$ und/oder der Pulsamplitude U verstärkt die Kontraktionskraft des Muskels über eine Zunahme der aktivierten Motoreinheiten (Spatial Recruitment).

Die Steuereingänge $c_1$ bis $c_n$ einer mit der Leistungsstufe 11 verbundenen Baugruppe 12 für die Anwahl von mindestens einer ausgangsseitig anschließbaren Elektrode $E_1$ bis $E_n$ mit definierter Zeitverzögerung sind ebenfalls mit entsprechenden Ausgängen des programmierbaren Decoders 4 verbunden. Damit kann eine der im Herzen vorliegenden vergleichbare Reizleitung im Skelettmuskel nachgebildet und/oder bei gleichzeitiger Ansteuerung die Stimulationsintensität erhöht werden. Für jede Elektrode $E_1$ bis $E_n$ ist in der Baugruppe 12 ein Ausgangsnetzwerk mit einer Schutzbeschaltung vorgesehen.

Die elektrischen Signale des Herzens werden von einer - in der Figur 1 nicht dargestellten - an der Wand des rechten Ventrikels intramural angeordneten Sensorelektrode empfangen und gegebenenfalls, wenn die Herzfrequenz unter einen programmierten Wert abfällt, wird der Herzmuskel über die gleiche Elektrode stimuliert.

Wenn aber die Herzfrequenz einen bestimmten vorprogrammierbaren Wert übersteigt, werden die Elektroden des Muskelstimulationskanals - gegebenenfalls nach Ablauf einer programmierbaren Verzögerungszeit - aktiviert, um einen Burst-Impuls über mindestens zwei Elektroden an den Muskulus latissimus dorsi (LDM) abzugeben. Die Burstdauer $T_B$ bestimmt die Dauer der Muskelkontraktion.

Sobald die Herzfrequenz aber einen weiteren vorprogrammierten Wert überschreitet, wird das Verhältnis von Herzaktion zur Muskelstimulationsanzahl bei gegebenem Stimulationsmodus m automatisch verändert. Das Verhältnis m von Herzkontraktion zu Muskelkontraktion ist von dem über die programmierbare Schaltung zur Burstlängeneinstellung 9 eingestelltem Wert abhängig.

Die Stimulationselektroden jedes Muskelstimulationskanals bestehen aus einem mit flexiblem Platin/Iridium-Nitrid beschichteten Draht mit einer extramuskulär so angeordneten Silikonisolierung, daß keine leitenden Elektrodenteile extramuskulär verbleiben. Sie können perineural, vorzugsweise aber intramuskulär in direkter Nachbarschaft zu den Nervenästen positioniert werden, indem ein an dem distalen Ende jeder Elektrode befestigter, nicht resorbierbarer Faden mit gebogener, zur Durchstechung des Muskels vorgesehener, Nadel durch den Muskel durchgezogen wird. Nachdem die Elektrode plaziert ist, wird die Isolierung bis auf das Muskelepimysium geschoben und dort fixiert. Eine als Kathode dienende Elektrode wird im Bereich der proximalen Verzweigung des Nervus thoraco dorsalis plaziert und die als Anode dienende Elektrode wenige Zentimeter distal davon.

Mittels der Programmiereinheit 3 ist über die Zuordnung eines Faktors n zur repräsentativen Herzrate HR die Anzahl der Einzelimpulse innerhalb eines Burst bzw. der Stimulationsmodus, das Pulsintervall $T_i$ = 1 / f mit f = 15 bis 40 Hz und die Burstdauer $T_B$ sowie die Leistung über die Impulsamplitude U und/oder Pulsbreite $t_p$ mittelbar veränderbar. Vorzugsweise werden als Parameter für eine Standardstimulation eingestellt: Impulsbreite $T_p$ = 210 µs, Impulsintervall $T_i$ = 30 ms, Impulsamplitude U = 5 V, Burstdauer $T_B$ = 185 ms bei einem Stimulationsmodus 1:1 bis 1:8.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch macht.

**Patentansprüche**

1.  Stimulationssystem für einen Skelettmuskel, in Ergänzung zu einer herkömmlichen Herzschrittmacherschaltung (2), wobei die Steuerung des mit dem Skelettmuskel verbundenen Stimulationsteils (1) durch den Schrittmacher (2) erfolgt, mit

    einer Signalverarbeitungsschaltung (1), die über eine Auswerteeinheit (4) und über ein einstellbaren Synchronisierteil (5) an die Ausgänge des Herzschrittmachers (2) angeschlossen ist, und einer in den Skelettmuskel implantierbaren Stimulationselektrode ($E_1$ bis $E_n$), wobei

    die Stimulationsintensität einer für die aktuelle körperliche Belastung bzw. den kardiovaskulären Bedarf repräsentativen Größe nachgeführt wird und

    **dadurch gekennzeichnet,**

    daß eine programmierbare Signalverarbeitungsschaltung (1) und eine programmierbare Auswerteeinheit (4) vorgesehen sind, wobei Stimulationsimpulse für mindestens zwei in dem Skelettmuskel implantierbare Stimulationselektroden ($E_1$ bis $E_n$) erzeugt werden, die aus den Ausgangssignalen des Herzschrittmachers (2) abgeleitet sind und daß deren reizwirksame Parameter Impulsbreite $T_p$ und Impulsamplitude U, bei

einem vorgegebenen Impulsintervall $T_i$ und einer mit dem Stimulationsmodus m eingestellten Burstdauer $T_B$, durch eine aus dem Zustand des autonomen Nervensystems direkt oder indirekt abgeleitete Größe, insbesonders Herzrate HR, entsprechend dem Bedarf automatisch eingestellt werden.

2. Stimulationssystem nach Anspruch 1, **dadurch gekennzeichnet,** daß die Stimulationsintensität über ihre Rate, ihre Amplitude ihre Dauer und/oder die Zahl der jeweils angesteuerten Stimulationselektroden der für die aktuelle körperliche Belastung bzw. den kardiovaskulären Bedarf repräsentativen Größe nachgeführt wird.

3. Stimulationssystem nach Anspruch 2, **dadurch gekennzeichnet,** daß die für die aktuelle körperliche Belastung bzw. den kardiovaskulären Bedarf repräsentative Größe bei intaktem Herzeigenrhytmus durch dessen Herzrate HR gebildet wird.

4. Stimulationssystem nach Anspruch 3, **dadurch gekennzeichnet,** daß in Abhängigkeit von der für die aktuelle körperliche Belastung oder den kardiovaskulären Bedarf repräsentativen Größe eine Untersetzung der Rate in einem von dieser Belastung abhängigen Verhältnis zur natürlichen - oder bei Ausbleiben des herzeigenen Rhythmus - zum durch den herkömmlichen Schrittmacher stimulierten Rhythmus erfolgt, wobei das Untersetzungsverhältnis mit zunehmender körperlicher Belastung bzw. kardiovaskulärem Bedarf bis zum Verhältnis 1:1 abnimmt.

5. Stimulationssystem nach einem der vorangehenden Ansprüche, **dadurch gekennzeichnet,** daß die für die aktuelle körperliche Belastung repräsentative Größe bei fehlendem Herzeigenrhythmus durch einen Sensor für die Aktivität des Patienten oder eine sekundäre für die Kreislaufbelastung repräsentative Größe, wie die Bluttmeperatur, die Blutsauerstoffsättigung oder den pH-Wert des Blutes erzeugt wird.

6. Stimulationssystem nach einem der vorangehenden Ansprüche, **dadurch gekennzeichnet,** daß die für den kardiovaskulären Bedarf repräsentative Größe durch eine von weiteren Einflußgrößen weitgehend unabhängige autonome Nervenaktivitätsgröße gebildet wird.

7. Stimulationssystem nach Anspruch 6, **dadurch gekennzeichnet,** daß es sich bei der autonomen Nervenaktivitätsgröße um die regionale effektive Steigung der Leitfähigkeit im rechten Ventrikel handelt.

8. Stimulationssytem nach Anspruch 7, **dadurch gekennzeichnet,** daß im Bereich der maximalen Leitfähigkeitsänderung während isovolumetrischer Kontraktion gemessen wird.

9. Stimulationssystem nach Anspruch 8, **dadurch gekennzeichnet,** daß ein Zeitfenster in den Bereich gelegt wird, in welchen bei unterschiedlicher Belastung jeweils die maximale Leitfähigkeitsänderung feststellbar ist.

10. Stimulationssystem nach einem der vorangehenden Ansprüche **dadurch gekennzeichnet,** daß die programmierbare Signalverarbeitungsschaltung (1) mit einer Programmiereinheit (3) verbunden ist und aus einer ein/ausschaltbaren Steuereinheit (6), die mit einer Spannungsverdopplerschaltung (7) und andererseits direkt und über eine programmierbare Schaltung zur Burstlängeneinstellung (9) indirekt mit einem Burst-Timer (8) verbunden ist, einem programmierbaren Start-Stop-Oszillator (10) und einer mit der Spannungsverdopplerschaltung (7) verbundenen programmierbaren Leistungsstufe (11) besteht, wobei das Verhältnis m von Herzaktion zu Muskelkontraktionsanzahl über eine an mindestens einem Steuereingang des Burst-Timers (8) angeschlossene, von der Programmiereinheit (3) über Programmiereingänge ($a_1$ bis $a_n$) programmierbare, Schaltung zur Burstlängeneinstellung (9) einstellbar ist, daß das an mindestens einem Eingang (d) zur Synchronisation eines Burst-Timers (8) angeschlossene einstellbare Synchronisierteil (5) von der programmierbaren Auswerteeinheit (4) ab einer definierten Herzrate HR so beaufschlagt wird, daß ein Burst-Impuls erzeugt wird, wobei, wenn die Herzfrequenz bzw. Herzrate HR einen weiteren vorprogrammierten Wert überschreitet, jeweils das Verhältnis von Herzaktion zur Muskelstimulationsanzahl bei gegebenen Stimulationsmodus m automatisch über eine von der programmierbaren Auswerteeinheit (4) vorgegebene Zahl n verändert wird.

11. Stimulationssystem nach Anspruch 10, **dadurch gekennzeichnet,** daß der Ausgang des Burst-Timers (8) mit dem Steuereingang des Start-Stop-Oszillators (10) verbunden ist, der entsprechend dem von den Schaltungen (4) und (9) vorgegebenen Stimulationsmodus m, während der Burstdauer $T_B$, kurze Impulse mit einer Zeitdauer von $T_p = 0,2$ bis 1 ms bei einer Erregungsfrequenz f = 15 bis 40 Hz abgibt, und daß eine Veränderung des Impulsintervalls $T_i = 1/f$ über mit der Programmiereinheit (3) verbundene Programmiereingänge ($i_1$ bis $i_n$) vornehmbar ist.

12. Stimulationssystem nach einem der vorangehenden Ansprüche, **dadurch gekennzeichnet,** daß die programmierbare Auswerteschaltung (4) an mindestens einem Steuereingang ($b_1$ bis $b_n$) einer Leistungsstufe (11) zur Verstärkung der Leistung der Ausgangsimpulse des Start-Stop-Oszillators (10), in Abhängigkeit von einer über die Steuereingänge ($b_1$ bis $b_n$) automatisch vorgenommenen und gleichzeitig über die Programmiereinheit (3) programmierbaren Einstellung, angeschlossen ist.

13. Stimulationssystem nach Anspruch 10, **dadurch gekennzeichnet,** daß die Steuereingänge ($c_1$ bis $c_n$) einer mit der Leistungsstufe (11) verbundenen Baugruppe (12) für die Anwahl von mindestens einer ausgangsseitig anschließbaren Stimulationselektrode ($E_1$ bis $E_n$) mit definierter Zeitverzögerung, ebenfalls mit entsprechenden Ausgängen der programmierbaren Auswerteschaltung (4) verbunden sind.

14. Stimulationssystem nach Anspruch 13, **dadurch gekennzeichnet,** daß für jede Stimulationselektrode ($E_1$ bis $E_n$) in der Baugruppe (12) ein Ausgangsnetzwerk mit einer Schutzbeschaltung vorgesehen ist.

15. Stimulationssystem nach einem der vorangehenden Ansprüche, **dadurch gekennzeichnet,** daß die programmierbare Auswerteschaltung (4) ein Decoder und das einstellbare Synchronisierteil (5) ein Dividierer ist.

16. Stimulationssystem nach Anspruch 10, **dadurch gekennzeichnet,** daß die Stimulationselektroden aus flexiblem, mit Platin/Iridium-Nitrid beschichtetem Draht bestehen und mit einer extramuskulär angeordneten Polyurethan-Isolierung versehen sind und daß ein an dem distalem Ende jeder Elektrode befestigter nicht resorbierbaren Faden mit gebogener, zur Durchstechung des Muskels vorgesehener, Nadel vorgesehen ist.

17. Stimulationssystem nach einem der vorangehenden Ansprüche, **dadurch gekennzeichnet,** daß mittels der Programmiereinheit (3) über die Zuordnung eines Faktors n zur repräsentativen Herzrate HR die Anzahl der Einzelimpulse innerhalb eines Burst bzw. der Stimulationsmodus, das Pulsintervall $T_i = 1/f$ mit f = 15 bis 40 Hz und die Burstdauer $T_B$ sowie die Leistung über die Impulsamplitude U und/oder Pulsbreite $t_p$ mittelbar veränderbar ist.

## Claims

1. A stimulation system for a skeletal muscle to complement a conventional cardiac pacemaker circuit (2), wherein the stimulation member (1) joined to the skeletal muscle is controlled by the pacemaker (2), comprising a signal-processing circuit (1), which is connected to the outputs of the pacemaker (2) by means of an evaluating unit (4) and by means of an adjustable synchronising member (5), and further comprising a stimulation electrode ($E_1$ to $E_n$) which can be implanted in the skeletal muscle, the stimulation intensity being controlled by a value representing the current physical strain or the cardiovascular requirement, and characterised in that a programmable signal-processing circuit (1) and a programmable evaluating unit (4) are provided, stimulation pulses derived from the output signals of the cardiac pacemaker (2) being generated for at least two stimulation electrodes ($E_1$ to $E_n$) which can be implanted in the skeletal muscle, and that their stimulation effect parameters, pulse width $T_p$ and pulse amplitude U, with a predetermined pulse interval $T_i$ and a burst duration $T_B$ adjusted with the stimulation mode $\underline{m}$, are automatically adjusted as required, by a value derived directly or indirectly from the state of the autonomic nervous system, particularly the heart rate HR.

2. The stimulation system of claim 1, characterised in that the stimulation intensity is controlled, in its rate, amplitude, duration and/or the number of stimulation electrodes activated in any given case, by the value representing the current physical strain or the cardiovascular requirement.

3. The stimulation system of claim 2, characterised in that the value representing the current physical strain or the cardiovascular requirement is formed by the heart rate HR when the heart's own rhythm is sound.

4. The stimulation system of claim 3, characterised in that, dependent on the value representing the current physical strain or the cardiovascular requirement, there results a reduction of the rate in a strain dependent ratio to the natural rhythm or - in the absence of the heart's own rhythm - to the rhythm stimulated by the conventional pacemaker, the reduction ratio decreasing with an increase in physical strain or cardiovascular requirement up to a ratio of 1 : 1.

5. The stimulation system of any of the preceding claims, characterised in that in the absence of the heart's own rhythm the value representing the current physical strain is produced by a sensor of the patient's activity or by a secondary value representing the strain on the circulation, such as the blood temperature, blood oxygen saturation or the pH of the blood.

6. The stimulation system of any of the preceding claims, characterised in that the value representing the cardiovascular requirement is formed by an autonomic nervous activity value which is substantially independent of other influences.

7. The stimulation system of claim 6, characterised in that the autonomic nervous activity value is the regional effective gradient in the conductivity in the right ventricle.

8. The stimulation system of claim 7, characterised in that the measurement is taken within the region of maximum change of conductivity during isovolumetric contraction.

9. The stimulation system of claim 8, characterised in that a time window is placed in the region in which, with differing strain, the maximum change in conductivity can be found in each case.

10. The stimulation system of any of the preceding claims, characterised in that the programmable signal processing circuit (1) is connected to a programming unit (3), comprises a control unit (6) which can be switched on and off and which is connected to a voltage doubling circuit (7) and at the other end, directly and indirectly via a programmable circuit (9) for burst length adjustment, to a burst timer (8), and further comprises a programmable start-stop oscillator (10) and a programmable power stage (11) connected to the voltage doubling circuit (7), the ratio $\underline{m}$ of heart action to the number of muscle contractions being adjustable by means of a burst length adjustment circuit (9) which is connected to at least one control input of the burst timer (8) and can be programmed by the programming unit (3) via programming inputs ($a_1$ to $a_n$), and that the adjustable synchronising member (5), which is connected to at least one input (d) for synchronisation of a burst timer (8), is acted on by the programmable evaluation unit (4) from a defined heart rate HR so that a burst pulse is generated, the ratio of heart action to the number of muscle stimulations in a given stimulation mode $\underline{m}$ being automatically changed by a number $\underline{n}$ predetermined by the programmable evaluation unit (4) whenever the heart frequency or heart rate HR exceeds a further pre-programmed value.

11. The stimulation system of claim 10, characterised in that the output of the burst timer (8) is connected to the control input of the start-stop oscillator (10) which, in accordance with the stimulation mode $\underline{m}$ predetermined by the circuits (4) and (9), transmits short pulses with a duration of $T_p = 0.2$ to 1 ms at an excitation frequency $\underline{f}$ of 15 to 40 Hz for the duration of the burst $T_B$, and that the pulse interval $T_i = 1/f$ may be changed by means of programming inputs ($i_1$ to $i_n$) connected to the programming unit (3).

12. The stimulation system of any of the preceding claims, characterised in that the programmable evaluation circuit (4) is connected to at least one control input ($b_1$ to $b_n$) of a power stage (11) for amplifying the power of the output pulses of the start-stop oscillator (10), dependent on a setting which is made automatically by means of the control inputs ($b_1$ to $b_n$) and which is simultaneously programmable by means of the programming unit (3).

13. The stimulation system of claim 10, characterised in that the control inputs ($c_1$ to $c_n$) of a module (12) connected to the power stage (11), for selecting at least one stimulation electrode ($E_1$ to $E_n$) with a defined time delay, which can be connected at the output side, are also connected to corresponding outputs of the programmable evaluation circuit (4).

14. The stimulation system of claim 13, characterised in that an output network with a protective circuit is provided for every stimulation electrode ($E_1$ to $E_n$) in the module (12).

15. The stimulation system of any of the preceding claims, characterised in that the programmable evaluation circuit (4) is a decoder and the adjustable synchronising member (5) is a divider.

16. The stimulation system of claim 10, characterised in that the stimulation electrodes are made of flexible wire coated with platinum/iridium nitride and are provided with polyurethane insulation arranged outside the muscle, and that a non-resorbable thread fixed to the distal end of each electrode is provided with a curved pin designed to pierce the muscle.

17. The stimulation system of any of the preceding claims, characterised in that, by means of the programming unit (3), by allocation of a factor $\underline{n}$ to the representative heart rate HR, the number of individual pulses within a burst or the stimulation mode, the pulse interval $T_i = 1/f$ with f = 15 to 40 Hz, the burst duration $T_B$ and the output are indirectly variable by means of the pulse amplitude U and/or pulse width $t_p$.

## Revendications

1. Système de stimulation pour un muscle du squelette, en complément à un circuit de stimulateur cardiaque (2) conventionnel, dans lequel la commande de la partie de stimulation (1) reliée au muscle du squelette s'effectue par le stimulateur cardiaque (2), système qui comprend un circuit de traitement de signal (1) raccordé à travers une unité d'exploitation (4) et une partie de synchronisation (5) réglable aux sorties du stimulateur cardiaque (2), ainsi qu'une électrode de stimulation ($E_1 - E_n$) implantable dans le muscle du squelette, et dans lequel l'intensité de stimulation est asservie à une grandeur représentative de la charge corporelle actuelle ou des besoins cardio-vasculaires,
caractérisé en ce

qu'un circuit de traitement de signal programmable (1) et une unité d'exploitation programmable (4) sont prévus,

avec génération d'impulsions de stimulation pour au moins deux électrodes de stimulation ($E_1$ - $E_n$) implantables dans le muscle du squelette, impulsions qui sont dérivées des signaux de sortie du stimulateur cardiaque (2),

et que les paramètres à activité excitatrice de ces impulsions, formés par la largeur ou durée d'impulsion $T_p$ et l'amplitude d'impulsion U, sont réglés automatiquement selon les besoins, pour un intervalle d'impulsion $T_i$ préfixé et une durée de salve $T_B$ réglée par le mode de stimulation m, au moyen d'une grandeur dérivée directement ou indirectement de l'état du système nerveux autonome, en particulier au moyen de la fréquence cardiaque HR.

2. Système de stimulation selon la revendication 1, caractérisé en ce que l'intensité de stimulation est asservie à la grandeur représentative de la charge corporelle actuelle ou des besoins cardio-vasculaires par sa fréquence, son amplitude, sa durée et/ou le nombre d'électrodes de stimulation commandées chaque fois.

3. Système de stimulation selon la revendication 2, caractérisé en ce que la grandeur représentative de la charge corporelle actuelle ou des besoins cardio-vasculaires est formée par la fréquence cardiaque HR du sujet lorsque le rythme cardiaque propre est intact.

4. Système de stimulation selon la revendication 3, caractérisé en ce que, en fonction de la grandeur représentative de la charge corporelle actuelle ou des besoins cardio-vasculaires, la fréquence est démultipliée, suivant un rapport à la fréquence naturelle dépendant de cette charge - ou, en cas d'absence du rythme cardiaque propre - suivant un rapport au rythme stimulé par le stimulateur cardiaque conventionnel, le rapport de démultiplication diminuant à mesure que la charge corporelle ou les besoins cardio-vasculaires augmente(nt), jusqu'à atteindre la valeur 1:1.

5. Système de stimulation selon une des revendications précédentes, caractérisé en ce que la grandeur représentative de la charge corporelle actuelle est générée, en cas d'absence du rythme cardiaque propre, par un capteur de l'activité du patient ou par une grandeur secondaire représentative de la charge du système circulatoire, telle que la température sanguine, la saturation en oxygène du sang ou la valeur pH du sang.

6. Système de stimulation selon une des revendications précédentes, caractérisé en ce que la grandeur représentative des besoins cardio-vasculaires est formée par une grandeur d'activité nerveuse autonome qui est pratiquement indépendante d'autres grandeurs d'influence.

7. Système de stimulation selon la revendication 6, caractérisé en ce que la grandeur d'activité nerveuse autonome est l'augmentation régionale effective de la conductance dans le ventricule droit.

8. Système de stimulation selon la revendication 7, caractérisé en ce que la mesure s'effectue dans la plage de variation maximale de la conductance pendant une contraction isovolumétrique.

9. Système de stimulation selon la revendication 8, caractérisé en ce qu'une fenêtre de temps est placée dans la plage où la variation maximale de conductance peut être constatée chaque fois sous des charges différentes.

10. Système de stimulation selon une des revendications précédentes, caractérisé en ce que le circuit de traitement de signal (1) programmable est relié à une unité de programmation (3) et composé d'une unité de commande (6) pouvant être enclenchée/arrêtée, qui est reliée d'une part à un circuit doubleur de tension (7) et d'autre part directement, de même qu'indirectement à travers un circuit (9) programmable pour le réglage de la longueur de salve, à un synchroniseur de salve (8), d'un oscillateur arythmique (10) programmable et d'un étage de puissance (11) programmable relié au circuit doubleur de tension (7), le rapport m de l'action cardiaque au nombre de contractions musculaires étant réglable par le circuit (9) pour le réglage de la longueur de salve, lequel circuit est raccordé à au moins une entrée de commande du synchroniseur de salve (8) et programmable par l'unité de programmation (3) à travers des entrées de programmation ($a_1$ - $a_n$), de manière que la partie de synchronisation (5) réglable, raccordée à au moins une entrée (d) pour la synchronisation du synchroniseur de salve (8), soit actionnée par l'unité d'exploitation (4) programmable à partir d'une fréquence cardiaque HR définie, de telle sorte qu'une impulsion de salve est générée, tandis que, si la fréquence cardiaque HR dépasse une autre valeur programmée préalablement, le rapport de l'action cardiaque au nombre de stimulations musculaires pour un mode de stimulation m donné, est automatiquement changé à l'aide d'un nombre n préfixé par l'unité d'exploitation (4) programmable.

11. Système de stimulation selon la revendication 10, caractérisé en ce que la sortie du synchroniseur de salve (8) est connectée à l'entrée de commande de l'oscillateur arythmique (10), lequel délivre pendant la durée de salve $T_B$, en conformité avec le mode de stimulation m préfixé par l'unité d'exploitation (4) et le circuit de réglage (9), de courtes impulsions d'une durée $T_p$ = 0,2 à 1 ms à une fréquence d'excitation f = 15 à 40 Hz, et que l'intervalle d'impulsion $T_i$ = 1/f peut être changé à travers les entrées de programmation ($i_1$ - $i_n$) reliées à l'unité de programmation (3).

12. Système de stimulation selon une des revendications précédentes, caractérisé en ce que le circuit d'exploitation (4) programmable est raccordé à au moins une entrée de commande ($b_1$ - $b_n$) d'un étage de puissance (11) destiné à amplifier la puissance des impulsions de sortie de l'oscillateur arythmique (10), en fonction d'un réglage effectué automatiquement à travers les entrées de commande ($b_1$ - $b_n$), et simultanément programmable au moyen de l'unité de programmation (3).

13. Système de stimulation selon la revendication 10, caractérisé en ce que les entrées de commande ($c_1$ - $c_n$) d'un module (12) relié à l'étage de puissance (11) et servant à la sélection avec un retard défini d'au moins une électrode de stimulation ($E_1$ - $E_n$) pouvant être raccordée à la sortie, sont connectées également à des sorties correspondantes du circuit d'exploitation (4) programmable.

14. Système de stimulation selon la revendication 13, caractérisé en ce qu'un réseau de sortie avec un montage de protection sont prévus dans le module (12) pour chaque électrode de stimulation ($E_1$ - $E_n$).

15. Système de stimulation selon une des revendications précédentes, caractérisé en ce que le circuit d'exploitation (4) programmable est un décodeur et la partie de synchronisation (5) réglable est un diviseur.

16. Système de stimulation selon la revendication 10, caractérisé en ce que les électrodes de stimulation sont faites d'un fil métallique flexible revêtu de platine/nitrure d'iridium et pourvues d'une isolation extramusculaire en polyuréthanne, et que chaque électrode est pourvue d'un fil non résorbable fixé à son extrémité distale et dont le bout est muni d'une aiguille courbe prévue pour transpercer le muscle.

17. Système de stimulation selon une des revendications précédentes, caractérisée en ce que, au moyen de l'unité de programmation (3), on peut changer indirectement, par la coordination d'un facteur n à la fréquence cardiaque HR représentative, le nombre d'impulsions individuelles à l'intérieur d'une salve ou le mode de stimulation, l'intervalle d'impulsion $T_i$ = 1/f , où f = 15 à 40 Hz, et la durée de salve $T_B$, ainsi que, par l'amplitude d'impulsion U et/ou la largeur ou durée d'impulsion $t_p$, la puissance.

Fig. 1

Fig. 2